# EUROPEAN PATENT SPECIFICATION

(11) **EP 4 070 840 B1**
(45) Date of publication and mention of the grant of the patent: **16.07.2025**
(21) Application number: 19955050.0
(22) Date of filing: 03.12.2019
(51) Int. Cl.: A61M 16/01, A61M 16/06, A61M 16/12

(54) **PATIENT VENTILATION MONITORING APPARATUS AND VENTILATION MONITORING METHOD THEREFOR**
PATIENTENBEATMUNGSÜBERWACHUNGSGERÄT UND BEATMUNGSÜBERWACHUNGSVERFAHREN DAFÜR
APPAREIL DE SURVEILLANCE DE VENTILATION DE PATIENT ET PROCÉDÉ DE SURVEILLANCE DE VENTILATION ASSOCIÉ

(43) Date of publication of application: 12.10.2022
(73) Proprietor: Shenzhen Mindray Bio-Medical Electronics Co., Ltd., Shenzhen, Guangdong 518057 (CN)
(72) Inventor: GUO, Jing, Shenzhen, Guangdong 518057 (CN); HUANG, Chenghua, Shenzhen, Guangdong 518057 (CN); HUANG, Jiping, Shenzhen, Guangdong 518057 (CN)
(74) Representative: KIPA AB
(86) International application number: PCT/CN2019/122794
(87) International publication number: WO 2021/109001

(56) References cited:
- WO-A1-2007/082384
- WO-A1-2011/157855
- WO-A1-2018/153964
- WO-A1-2018/153964
- CN-A- 1 373 670
- CN-A- 103 002 802
- CN-A- 103 893 866
- US-A1- 2003 078 512
- US-A1- 2019 231 202

## Description

### TECHNICAL FIELD

The invention relates to the technical field of medical devices, and more particularly to a patient ventilation monitoring apparatus, an anesthetic ventilation device and and an apparatus for implementing patient ventilation montitoring on an anesthetic ventilation device according to claims 1 to 15. The terms "disclosure" and "embodiment" cover only to the invention, when they are covered by the claims.

### BACKGROUND

In general anesthesia surgery, as patients do not breathe spontaneously, they often rely on anesthesia ventilation apparatus to provide respiratory support. According to clinical statistics, about 85%-90% of patients have the risks of alveolar collapse and atelectasis, resulting in decreased pulmonary compliance, worsening oxygenation, increased dead space, decreased functional residual capacity, and changed hemodynamic. Severe atelectasis prolongs the treatment time of postoperative recovery, increases the incidence rate of postoperative pulmonary infection and other complications, prolongs the hospitalization period of patients, and increases the treatment cost. Clinical studies show that the implementation of lung-protective ventilation can significantly reduce the probability of atelectasis. At present, lung-protective ventilation strategy includes the following aspects.

Firstly, an appropriate tidal volume is preset to avoid excessive pulmonary volume at the inhalation end, thus avoiding the barotrauma and volutrauma to patients and reducing the incidence rate of postoperative pulmonary complications. In order to achieve an appropriate pulmonary volume at the inhalation end, the anesthesiologists need to accurately preset the inspiratory pressure or inspiratory tidal volume. How to preset a safe inspiratory pressure or inspiratory tidal volume is a clinical difficulty. The same inspiratory pressure has different safeties for patients with different pulmonary status. At present, clinical anesthesiologists can only guarantee that the airway pressure at the inspiratory end is not higher than a certain threshold according to experience. However, for patients with certain pulmonary diseases, such as thoracic restriction, the alveolar pressure at the conventional inspiratory end may be too low, which may result in insufficient ventilation. For some patients with strong spontaneous respiration, the alveolar pressure at the conventional inspiratory end may be too high, which may lead to the risk of excessive alveolar inflation.

Secondly, an appropriate positive expiratory end pressure (PEEP) is arranged to maintain the open state of alveolus. This is especially important for patients undergoing laparoscopic surgery, one-lung ventilation, obesity, cardiopulmonary dysfunction and poor oxygenation. But for different patients, the most appropriate PEEP values are different. How to set the best PEEP is a clinical difficulty. If PEEP is to be accurately preset, the most standard manner is to rely on CT and other imaging apparatuses. However, this method is not easy to implement in the surgery process, and it is not possible to be widely used. At present, clinical anesthesiologists can only roughly set PEEP value according to experience, which results in the risks that PEEP value is either too high or too low.

Thirdly, the patients with atelectasis are treated with pulmonary re-expansion operation to recover the open state of alveolus. Pulmonary re-expansion is an important aspect of pulmonary protection strategy, but only pulmonary re-expansion cannot maintain the open state of alveolus for a long time. It is also necessary to set up a reasonable PEEP.

US2019231202 teaches a system for recording breathing efforts of a patient, in which the transpulmonary pressure is calculated based on the respiratory pressure, which predetermined or specified by the respirator according to the change of the esophageal pressure, and the esophageal pressure, which is determined by the sensor. Furthermore, the respiratory pressure is controlled based on the changes of the esophageal pressure. Such obtained transpulmonary pressure may not be accurate and such control of respiratory pressure may not use sufficient information about the patient, so cannot be effectively and accurately.

### SUMMARY

This disclosure mainly provides a patient ventilation monitoring apparatus and a ventilation monitoring method therefor, wherein the patient ventilation monitoring apparatus can be used in an anesthetic device.

According to a first aspect, an embodiment provides a patient ventilation monitoring apparatus, which including:
a sensor interface, which is configured to receive an airway pressure value of the patient, which is monitored by a first pressure sensor and an esophageal pressure value of the patient, which monitored by a second pressure sensor;
a processor, which is in signal connection with the sensor interface and configured to receive the airway pressure value and the esophageal pressure value respectively, to obtain a transpulmonary pressure through calculating a difference between the airway pressure value and the esophageal pressure value, and guide mechanical ventilation according to the transpulmonary pressure.

According to a second aspect, an embodiment provides an anesthetic ventilation device, which including:
a gas source interface, which is connected with an external gas source;
a respiratory line, which is configured to connect the gas source interface with a respiratory system of patient, to input gas which is provided by the gas source to the patient and to receive exhaled gas of the patient;
an anesthetic drug output apparatus, which is configured to mix stored anesthetic drug with input gas, and to output the gas which is mixed with the anesthetic drug to the respiratory line;
a respiratory assistance apparatus, which is configured to provide respiratory support power for controlling, the gas which is provided by the gas source and the gas, which is mixed with the anesthetic drug and outputted by the anesthetic drug output apparatus, to be outputted to the patient; or for recycling the exhaled gas of the patient; or for discharging the exhaled gas of the patient to external environment; wherein the respiratory assistance apparatus includes a computer-controlled ventilation module and/or a manual ventilation module; and
any patient ventilation monitoring apparatus which is discussed above.

According to a third aspect, an embodiment provides a patient ventilation monitoring method, which including:
acquiring an airway pressure value and an esophageal pressure value of a patient;
obtaining a transpulmonary pressure through calculating a difference between the airway pressure value and the esophageal pressure value, and
guiding mechanical ventilation according to the transpulmonary pressure.

According to a fourth aspect, an embodiment provides an apparatus for implementing patient ventilation monitoring on an anesthetic ventilation device, including:
a memory, which is configured to store a program;
a processor, which is configured to implement the above patient ventilation monitoring method, by executing the program which is stored in the memory.

According to a fifth aspect, an embodiment provides a computer-readable storage medium on which a program is stored, wherein when the program is executed by a processor, the above patient ventilation monitoring method is implemented.

In the above embodiments, the ventilation monitoring apparatus can monitor the transpulmonary pressure, and guide various ventilation parameters and pulmonary re-expansion operation according to the transpulmonary pressure, thus safely and effectively implementing the mechanical ventilation in clinical surgery.

### BRIEF DESCRIPTION OF THE DRAWINGS

FIG. 1 is a structure and composition diagram of an anesthetic ventilation device (or anesthetic machine) of an embodiment.
FIG. 2 is a schematic diagram showing that a sensor of an embodiment acquires respiratory information of a patient.
FIG. 3 is a composition module diagram of an anesthetic ventilation device of an embodiment.
FIG. 4 is a general flow diagram for guiding mechanical ventilation according to transpulmonary pressure in an embodiment.
FIG. 5a is a flow diagram for guiding mechanical ventilation according to transpulmonary pressure at inspiratory end of an embodiment.
FIG. 5b is a flow diagram for guiding mechanical ventilation according to transpulmonary pressure at expiratory end of an embodiment.
FIG. 6 is a waveform of airway pressure and transpulmonary pressure of an embodiment.
FIG. 7 is a general flow diagram for guiding pulmonary re-expansion operation according to transpulmonary pressure of an embodiment.
FIG. 8 is a flow diagram for obtaining transpulmonary pressure effect evaluation of an embodiment.
FIG. 9 is a schematic diagram of a first transpulmonary pressure-volume cyclic graph of an embodiment.
FIG. 10 is a schematic diagram showing a first transpulmonary pressure-volume cyclic graph and a second transpulmonary pressure-volume cyclic graph, which are superimposed and displayed on a display interface in an embodiment; wherein the first transpulmonary pressure-volume cyclic graph and the second transpulmonary pressure-volume cyclic graph are obtained before and after pulmonary re-expansion operation which is implemented through a sustained inflation method.
FIG. 11 is a schematic diagram showing waveforms of transpulmonary pressures at expiratory end on a display interface in an embodiment, wherein the waveforms of transpulmonary pressures are obtained before and after pulmonary re-expansion operation, which is implemented through a PEEP incremental or PEEP decremental method.

### DESCRIPTION OF THE PREFERRED EMBODIMENTS

This disclosure is further described in detail below through specific embodiments in combination with the accompanying drawings. In different embodiments, similar elements adopt associated similar reference numbers. In the following embodiments, many details are described in order to make this disclosure better understood. However, one skilled in the art can easily recognize that some of the features can be omitted in different cases, or can be replaced by other elements, materials and methods. In some cases, some operations related to this disclosure are not shown or described in the description, in order to avoid the core part of this disclosure being inundated by too many descriptions. For one skilled in the art, it is not necessary to describe these related operations in detail, as they can fully understand the relevant operations according to the description in this disclosure and the general technical knowledge in the art.

In addition, the features, operations, or features described in the specification may be combined in any appropriate manner to form various embodiments. At the same time, the steps or actions in the method description can also be exchanged or adjusted in order in a manner obvious to one skilled in the art. Therefore, the various sequences in the description and the drawings are only for the purpose of clearly describing a certain embodiment, and do not mean that they are necessary sequences. Unless it is otherwise specified that one of the sequences must be followed.

The terms "first", "second", etc., in the specification are operable to distinguish different objects, rather than to describe a specific order. In addition, the terms "connection", and "linkage" mentioned in this application include direct and indirect connection (linkage), unless otherwise specified.

Human respiration refers to periodic rhythmic inhalation and exhalation of gas, absorption of oxygen and discharge of carbon dioxide, which are for gas exchange. From the microscopic level, respiration is characterized by the periodic and rhythmic expansion and contraction of the alveolus, and the direct drive force for the expansion and contraction of the alveolus is the pressure difference inside and outside the alveolus, which is called transpulmonary pressure in this disclosure. The transpulmonary pressure is equal to the alveolar pressure minus the intrathoracic pressure. The alveolar pressure can be indirectly replaced by airway pressure. When there is no flowing gas in the airway, the airway pressure is equal to the alveolar pressure. Intrathoracic pressure is difficult to measure. In clinical practice, a pressure sampling tube is inserted into the esophagus of the patient, and the esophageal pressure is usually used to instead intrathoracic pressure. In the embodiment of this disclosure, when the patient is under anesthesia, the transpulmonary pressure of the patient is detected and monitored, then the rhythmic movement of the alveolus is known by monitoring the transpulmonary pressure. That is, the air content of the alveolus of the patient is known, such that whether the patient has the risk of atelectasis can be monitored. In additional, the mechanical ventilation of the patient, such as the ventilation parameter setting or the operation of the pulmonary re-expansion operation, can be guided based this. In this way, the transpulmonary pressure at expiratory end is maintained to be greater than 0, and the alveolar is guaranteed to maintain open state for avoiding atelectasis.

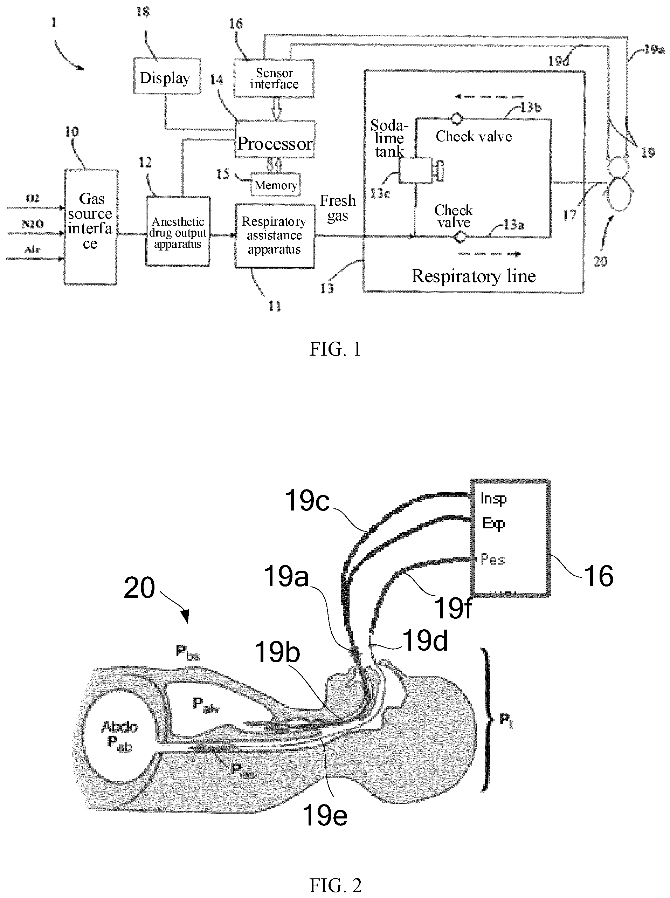

FIG. 1 is a structure and composition diagram of an anesthetic ventilation device (or anesthetic machine) of an embodiment. Referring FIG.1, the anesthetic machine includes a gas source interface 10, a respiratory assistance apparatus 11, an anesthetic drug output apparatus 12, a respiratory line 13, a processor 14, a memory 15, a sensor interface 16 and a display 18.

The gas source interface 10 is configured to connect with the gas source (not shown), which is configured to provide a gas. The gas can usually be oxygen, nitrous oxide (laughing gas), air and so on. In some embodiments, the gas source can be a compressed gas cylinder or a central gas supply source, which supplies the gas to the anesthetic machine through the gas source interface 10. The types of gas include oxygen O₂, nitrous oxide N₂O, air, etc. The gas source interface 10 can include conventional components, such as pressure gauge, pressure regulator, flowmeter, pressure reducing valve and N₂O-O₂ proportional control protection device, which are respectively configured to control the flow of various gases (such as oxygen, laughing gas and air). The gas, which is inputted from the gas source interface 10, enters the respiratory line 13 and forms a mixed gas with the original gas in the respiratory line 13.

The respiratory assistance apparatus 11 is configured to provide power for the non-spontaneous respiration, maintain the airway unblocked, drive the gas which is inputted from the gas source interface 10 and the mixed gas which is in the respiratory line 13 to the respiratory system of the patient, and drain the exhaled gas of the patient into the respiratory line 13, so as to improve ventilation and oxygenation and prevent the body of the patient from hypoxia and carbon dioxide accumulation. In a specific embodiment, the respiratory assistance apparatus 11 usually includes a machine-controlled ventilation module, and the gas duct of the machine-controlled ventilation module is connected with the respiratory line 13. During the anesthesia maintenance stage of the surgery or when the patient does not resume spontaneous respiration, the machine-controlled ventilation module is configured to provide the patient with respiratory power. In some embodiments, the respiratory assistance apparatus 11 also includes a manual ventilation module, and the gas duct of the manual ventilation module is connected with the respiratory line 13. During the induction stage before intubation, the manual ventilation module is usually configured to assist the patient in respiration. When the respiratory assistance apparatus 11 includes both machine-controlled ventilation module and manual ventilation module, the machine-controlled ventilation mode and the manual ventilation mode can be switched through a machine-controlled or manual switch (such as a three-way valve), so as to connect the machine-controlled ventilation module or manual ventilation module with the respiratory line 13 for controlling the respiration of the patient. Those skilled in the art should understand that the anesthetic machine can only include a machine-controlled ventilation module or a manual ventilation module according to specific requirements.

The anesthetic drug output apparatus 12 is configured to provide anesthetic drugs. Generally, the anesthetic drugs are mixed into fresh air which is introduced by the gas source interface 10 in the form of gas and delivered to the respiratory line 13 together. In a specific embodiment, the anesthetic drug output apparatus 12 is realized by using an anesthetic drug volatilization tank. Anesthetic drugs are usually liquid and stored in the anesthetic drug volatilization tank. Optionally, the anesthetic drug volatilization tank can include a heating device to heat the anesthetic drug to volatilize and generate anesthetic steam. The anesthetic drug output apparatus 12 is connected with the pipeline of the gas source interface 10. The anesthetic steam is mixed with the fresh air which is introduced from the gas source interface 10 and then transmitted to the respiratory line 13 together.

The respiratory line 13 includes an inspiratory line 13a, an expiratory line 13b and a soda-lime tank 13c, wherein the inspiratory line 13a and the expiratory line 13b are connected to form a closed loop. The soda-lime tank 13c is arranged at expiratory line 13b. The mixed gas of anesthetic steam and fresh air which is introduced from the gas source interface 10, is inputted from an inlet of the inspiratory line 13a and supplied to the patient 20 through the patient interface 17 arranged at an outlet of the inspiratory line 13a. The patient interface 17 may be a mask, a nasal intubation, or an endotracheal intubation. In a preferred embodiment, the inspiratory line 13a is provided with a check valve, which opens in the inspiratory phase and closes in the expiratory phase. The expiratory line 13b is also provided with a check valve, which closes in the inspiratory phase and opens in the expiratory phase. The inlet of the expiratory line 13b is connected with the patient interface 17. When the patient exhales, the exhaled gas enters the soda-lime tank 13c through the expiratory line 13b. The carbon dioxide in the exhaled gas is filtered by the substances in the soda-lime tank 13c, and the filtered gas is recycled into the inspiratory line 13a. In some embodiments, the respiratory line 13 is also provided with a flow sensor and/or a pressure sensor for detecting the gas flow and/or the pressure in the pipeline, respectively.

The sensor interface 16 is configured to receive various respiratory information of the patient sampled by the sensor, such as the airway pressure value and esophageal pressure value of the patient which are sampled by the sensor under the machine assisted ventilation state. Specifically, the sensor interface 16 is connected to signal output terminals of a first pressure sensor 19a and a second pressure sensor 19d, respectively. As shown in FIG.2, the first sampling tube 19b enters the trachea through the oral cavity. The first pressure sensor 19a is arranged inside the first sampling tube 19b to monitor the pressure in the trachea (i.e., airway pressure). The airway pressure is equal to the alveolar pressure. An airway pressure electrical signal which is outputted by the first pressure sensor 19a is transmitted to the sensor interface 16 through a first wire 19c. A second sampling tube 19e enters the esophagus through the nasal cavity, and the second pressure sensor 19d is arranged inside the second sampling tube 19e to monitor the pressure in the esophagus, which is equal to the intrathoracic pressure. Then the second pressure sensor 19d converts the sampled esophageal pressure into an electrical signal and transmits it to the sensor interface 16 through the second wire 19f. In one embodiment, the sensor interface 16 may simply function as a connector between the output terminal of the sensor output and a subsequent circuit (e.g, a processor) without processing the signals. The sensor interface 16 may also be integrated into the processor 14 as an interface of the processor 14 for accessing signals. In another embodiment, the sensor interface 16 may include an amplification circuit, a filter circuit and an A/D conversion circuit for amplifying, filtering and analog-to-digital conversion processing of the inputted analog signal, respectively. Of course, those skilled in the art should understand that the connection relationship among the amplification circuit, the filter circuit and the A/D conversion circuit can vary according to the specific design of the circuit, or a certain circuit can be reduced, for example, the amplification circuit or the filter circuit can be reduced, so as to reduce its corresponding functions. In addition, the sensor interface 16 can also access information sampled by other sensors of the respiratory line, such as flow information and/or pressure information.

As specific products, the first pressure sensor 19a, the first sampling tube 19b, the second pressure sensor 19d, and the second sampling tube 19e may be part of the anesthetic ventilation device 1, or may be peripheral accessories independent of the anesthetic ventilation device 1.

The memory 15 may be configured to store data or program, such as data sampled by each sensor, data generated by the processor through calculation, or image frames generated by the processor. The image frames may be 2D or 3D images. Optionally, the memory 15 may store a graphical user interface, one or more default image display settings, and programming instructions for the processor. The memory 15 may be a tangible and non-transient computer-readable medium, such as flash memory, RAM, ROM, EEPROM, and the like.

The processor 14 is configured to execute instructions or programs, control various control valves in the respiratory assistance apparatus 11, the gas source interface 10 and/or the respiratory line, or process the received data, generate the required calculation or determination results, or generate visual data or graphics, and output the visual data or graphics to the display 18 for displaying. In this embodiment, the processor 14 is in signal connection with the sensor interface and configured to calculate or generate waveforms according to the airway pressure value, the esophageal pressure value, the gas flow value in the respiratory line and/or the pressure value in the respiratory line. For example, the processor 14 calculates the transpulmonary pressure of the patient in real time according to the airway pressure value and the esophageal pressure value, for example, calculates the difference between the airway pressure value and the esophageal pressure value, and takes the difference between the airway pressure value and the esophageal pressure value as the transpulmonary pressure. The processor 14 then guides the mechanical ventilation according to the transpulmonary pressure which is calculated in real time. The operation of mechanical ventilation may include, for example, the ventilation parameter setting and pulmonary re-expansion operation. For example, the processor 14 obtains the transpulmonary pressure at inspiratory end and/or the transpulmonary pressure at expiratory end according to the monitored transpulmonary pressure, and guides the ventilation parameter setting, such as inspiratory pressure setting, tidal volume setting or positive expiratory end pressure setting, according to the transpulmonary pressure at inspiratory end and/or the transpulmonary pressure at expiratory end. For another example, the processor 14 obtains the transpulmonary pressure at expiratory end according to the monitored transpulmonary pressure, and guides the pulmonary re-expansion operation according to the transpulmonary pressure at expiratory end.

In order to facilitate the user (e.g., a doctor or medical staff) to view, in a specific embodiment, the processor also obtains a parameter waveform and/or a parameter monitoring value according to the airway pressure value and/or the esophageal pressure value, and outputs the parameter waveform and/or the parameter monitoring value to a display interface for display. The parameter waveform can include at least one of the airway pressure waveform, the esophageal pressure waveform and the transpulmonary pressure waveform that change with time. The parameter monitoring value may include at least one of the transpulmonary pressure at inspiratory end, the transpulmonary pressure at expiratory end and the transpulmonary pressure. In another embodiment, the processor also acquires the monitored pulmonary volume and generates a pressure-volume cyclic graph that changes with the respiratory cycle. The pressure-volume cyclic graph includes an esophageal pressure-volume cyclic graph and/or a transpulmonary pressure-volume cyclic graph. In a further embodiment, the processor outputs a first transpulmonary pressure-volume cyclic graph and a second transpulmonary pressure-volume cyclic graph to the display interface of the display for superimposed displaying after detecting that the pulmonary re-expansion operation is started. The first transpulmonary pressure-volume cyclic graph is the transpulmonary pressure-volume cyclic graph before the pulmonary re-expansion operation, and the second transpulmonary pressure-volume cyclic graph is the transpulmonary pressure-volume cyclic graph after the pulmonary re-expansion operation.

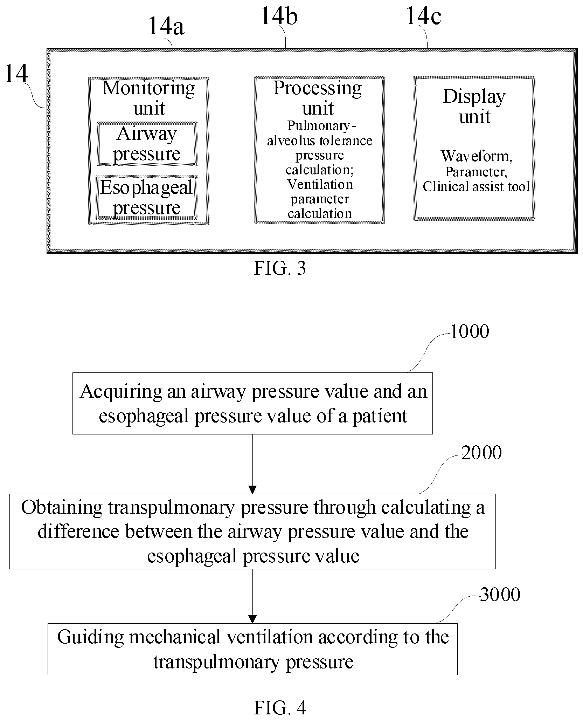

In a specific embodiment, as shown in FIG. 3, the processor 14 includes a monitoring unit 14a, a processing unit 14b and a display unit 14c. The monitoring unit 14a is configured to monitor the airway pressure and esophageal pressure, the processing unit 14b is configured to calculate the transpulmonary pressure according to the airway pressure and esophageal pressure which are outputted by the monitoring unit 14a, and to calculate the ventilation parameters (such as tidal volume and/or positive expiratory end pressure) according to a real-time transpulmonary pressure, and to obtain the parameter waveform and/or parameter monitoring value according to the airway pressure and/or esophageal pressure. The display unit 14c is configured to generate an image frame for displaying the waveform and/or parameter values according to various parameter waveforms and/or parameter monitoring values which are obtained by the processing unit 14b, for example, to generate image frames including a waveform showing that the transpulmonary pressure changes with time, image frames including a waveform showing that the gas flow changes with time, or to generate image frames containing visual respiratory parameters or ventilation parameter values.

Based on the above anesthetic ventilation device, various schemes for guiding mechanical ventilation according to transpulmonary pressure are described in combination with the flow diagram as follows.

FIG. 4 is a general flow diagram for guiding mechanical ventilation according to transpulmonary pressure in an embodiment, which includes following steps.

In step 1000, an airway pressure value and an esophageal pressure value of a patient are acquired. For example, when the patient is in the state of machine-assisted ventilation, the first sampling tube 19b arranged inside with the first pressure sensor 19a, enters into the trachea through the oral cavity to monitor the pressure in the trachea (i.e., airway pressure). The airway pressure is equal to the alveolar pressure. The airway pressure electrical signal which is outputted by the first pressure sensor 19a is transmitted to the sensor interface 16 through the first wire 19c. The second sampling tube 19e arranged inside with the second pressure sensor 19d, enters into the esophagus through the nasal cavity to monitor the pressure in the esophagus, wherein the esophageal pressure is equal to the intrathoracic pressure. Then the second pressure sensor 19d converts the sampled esophageal pressure into an electrical signal and transmits it to the sensor interface 16 through the second wire 19f.

In step 2000, a transpulmonary pressure is obtained through calculating a difference between the airway pressure value and the esophageal pressure value.

In order to facilitate the user (e.g., a doctor or medical staff) to view, the processor 14 can obtain the transpulmonary pressure waveform and/or the transpulmonary pressure monitoring value according to the monitored transpulmonary pressure. At the same time, the display unit 14c generates image frames which include a waveform showing that the transpulmonary pressure changes with time, image frames which include a waveform showing that the gas flow changes with time, and outputs the image frames to the display interface for display.

In addition to the real-time alveolar pressure, the processor 14 can also obtain other parameter waveforms and/or parameter monitoring values according to the airway pressure value and/or esophageal pressure value, and output the parameter waveforms and/or parameter monitoring values to the display interface for display. For example, the parameter waveforms can also include at least one of the airway pressure waveform, esophageal pressure waveform and the likes that change with time, the parameter monitoring values can also include the airway pressure value, esophageal pressure value and the likes that change with time.

In some embodiments, the processor 14 can also obtain the change trend of the corresponding transpulmonary pressure, airway pressure value and esophageal pressure value according to the airway pressure value and esophageal pressure value, and output the change trend to the display interface in the form of visual graphics for displaying.

In step 3000, mechanical ventilation is guided according to the transpulmonary pressure. The guidance on the mechanical ventilation includes the guidance on the ventilation parameter setting when the patient is in the state of machine assisted ventilation, the guidance on the ventilation parameter setting for pulmonary re-expansion operation and the guidance on the pulmonary re-expansion operation, and the effect evaluation of the pulmonary re-expansion operation. In specific implementation, the effect of pulmonary re-expansion can be evaluated by changes in physiological parameters before and after the pulmonary re-expansion operation. For example, the effect of pulmonary re-expansion can be evaluated according to changes in physiological parameters such as, compliance increase, tidal volume increase, plateau pressure decrease, or blood oxygen improvement.

The guiding principle on the ventilation parameter setting, according to the monitored transpulmonary pressure during machine-controlled ventilation, is as follows.

During machine-controlled ventilation, as the patient does not breathe spontaneously, improper ventilation parameter setting may damage the lungs of the patient. For example, if the inspiratory pressure or inspiratory tidal volume is set too large during ventilation, the pulmonary volume at inspiratory end, the alveolus of the patient is at risk of excessive expansion. If the positive expiratory end pressure is too small during ventilation, the alveolus cannot maintain a long-term open state, and there is a risk of alveolus collapse.

Because the direct drive force of alveolar expansion and contraction is the pressure difference inside and outside the alveolus, that is, the transpulmonary pressure. Accordingly, the transpulmonary pressure can indirectly indicate whether the alveolus is ventilated or not adequately ventilated. If the transpulmonary pressure is within the preset reasonable range, the occurrence of pulmonary injury, such as the reduction of the volume or gas content of one or more pulmonary segments or pulmonary lobes (atelectasis) can be avoided. For example, studies have shown that maintaining the transpulmonary pressure at expiratory end greater than 0 can ensure that the alveolus remains open. There is a certain functional relationship between the ventilation parameter and transpulmonary pressure. For example, the above inspiratory pressure, inspiratory tidal volume and positive expiratory end pressure are positively correlated with the transpulmonary pressure, respectively. Users can determine whether the current ventilation parameters need to be adjusted according to that whether the monitored transpulmonary pressure is within the preset reasonable range, and determine which direction the ventilation parameters need to be adjusted according to the functional relationship between transpulmonary pressure and ventilation parameters. For example, when the monitored transpulmonary pressure at inspiratory end is greater than the preset threshold, it means that the inspiratory pressure or inspiratory tidal volume is too large. The processor can directly decrease the parameter value of the inspiratory pressure or inspiratory tidal volume, or the user can manually decrease the parameter value of the inspiratory pressure or inspiratory tidal volume according to this.

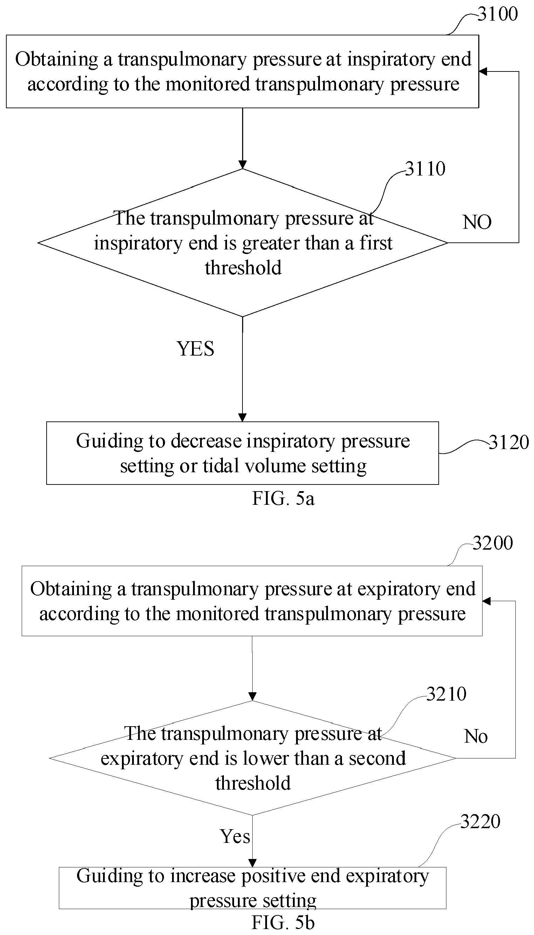

In one embodiment, as shown in FIG. 5a, which is a flow diagram for guiding mechanical ventilation according to transpulmonary pressure at inspiratory end of an embodiment.

Guiding mechanical ventilation according to the monitored transpulmonary pressure, in step 3000, includes following steps.

In step 3100, the transpulmonary pressure at inspiratory end in a current respiratory cycle is obtained according to the monitored transpulmonary pressure. The transpulmonary pressure at inspiratory end is the transpulmonary pressure which corresponds to end time of an inspiratory phase of the respiratory cycle, and the inspiratory phase is a time period for the patient to inhale. In the waveform which is generated in step 2000 and shows the transpulmonary pressure changes with time, the transpulmonary pressure which corresponds to the highest point of the wave peak in one respiratory cycle is the transpulmonary pressure at inspiratory end of this respiratory cycle.

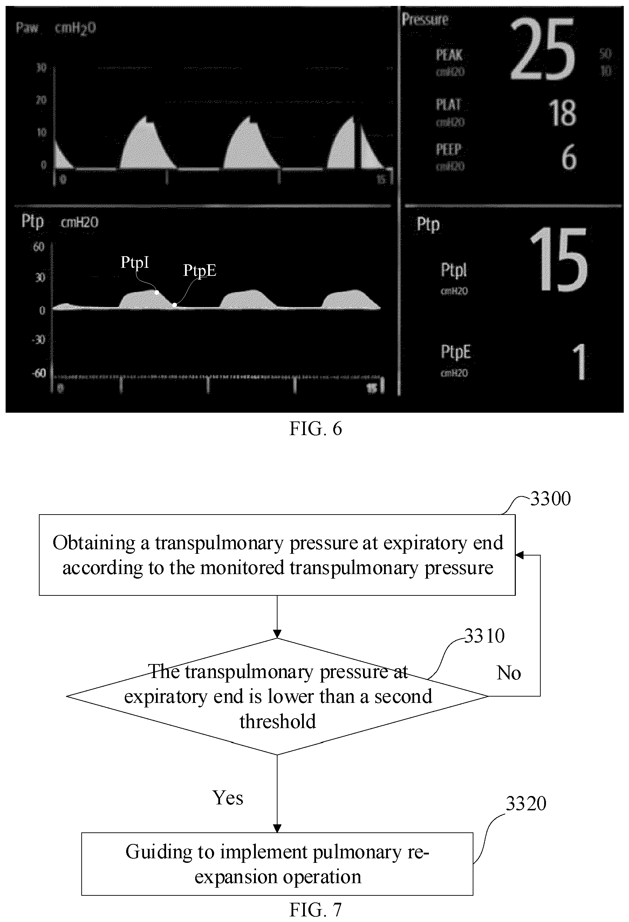

For example, FIG. 6 is a display interface showing the transpulmonary pressure in an embodiment. In this display interface, the left side includes a waveform of airway pressure (PAW) and a waveform of transpulmonary pressure (Ptp) from top to bottom. The current ventilation parameters can be obtained from the right column of the diagram. The peak value of airway pressure is 25cmH2O, the plateau pressure PLAT is 18cmH2O, the positive expiratory end pressure is 6cmH2O, and the transpulmonary pressure at inspiratory end PtpI is 15cmH2O.

In step 3110, whether the transpulmonary pressure at inspiratory end is greater than a first threshold, is determined. If it is greater than the first threshold, step 3120 is executed, otherwise, step 3100 is executed in the next respiratory cycle.

The first threshold is a preset parameter. For example, it can be a default empirical value in the program. This empirical value can depend on the clinical medical research and doctor experience. For example, the research shows that for patients with good physiological conditions, the transpulmonary pressure at inspiratory end, which is below 25cmH2O, can reduce the probability of pulmonary injury. Therefore, the first threshold can be 25 cmH2O. The first threshold can also be a value which is calculated by a formula according to other parameters in the program. In some embodiments, the first threshold may also allow the user to adjust according to the actual situation of the patient.

In step 3120, inspiratory pressure setting or inspiratory tidal volume setting is guided to be decreased. When the ventilation device is set in a volume mode, the tidal volume setting is guided according to the transpulmonary pressure at inspiratory end. When the ventilation device is set in a pressure mode, the inspiratory pressure setting is guided according to the transpulmonary pressure at inspiratory end.

When guiding the reduction of the inspiratory pressure setting or inspiratory tidal volume setting, the user can be guided by setting instructions. For example, an image frame containing characters, such as "please decrease inspiratory pressure setting or inspiratory tidal volume setting", is stored in the memory 15 in advance. When determining that the transpulmonary pressure at inspiratory end is greater than the first threshold, the processor 14 calls the image frame and inputs it to the display unit 14c to instruct the user. The user can manually operate it.

When guiding the reduction of the inspiratory pressure setting or inspiratory tidal volume setting, the user can be guided by directly changing the original setting. For example, each time determining that the transpulmonary pressure at inspiratory end is greater than the first threshold, the processor 14 controls the currently set inspiratory pressure or inspiratory tidal volume to decrease by a fixed value until the transpulmonary pressure at inspiratory end in the next respiratory cycle is not greater than the first threshold. For example, if the currently set inspiratory tidal volume is 10ml/kg, and the transpulmonary pressure at inspiratory end in the current respiratory cycle is greater than the first threshold, the processor 14 controls the inspiratory tidal volume to decrease by 1ml/kg, and continues to determine the relationship between the transpulmonary pressure at inspiratory end and the first threshold in the next respiratory cycle. If determining that the transpulmonary pressure at inspiratory end in the next respiratory cycle is still greater than the first threshold, the processor 14 continues to decrease the inspiratory tidal volume by 1ml/kg, until the transpulmonary pressure at inspiratory end in a certain respiratory cycle is not greater than the first threshold, and then maintains the inspiratory tidal volume setting in that respiratory cycle.

This example can effectively reduce the risk of excessive alveolar inflation by monitoring the transpulmonary pressure at inspiratory end and guiding the reduction of the inspiratory pressure setting or inspiratory tidal volume setting.

In another embodiment, as shown in FIG. 5b, a flow diagram for guiding mechanical ventilation according to transpulmonary pressure at expiratory end of an embodiment is disclosed. Guiding mechanical ventilation guided according to the monitored transpulmonary pressure, in step 3000, includes following steps.

In step 3200, the transpulmonary pressure at expiratory end in a current respiratory cycle is obtained according to the monitored transpulmonary pressure.

The transpulmonary pressure at expiratory end is the transpulmonary pressure which corresponds to end time of an expiratory phase of the respiratory cycle, and the expiratory phase is a time period for the patient to exhale. In the waveform which is generated in step 2000 and shows the transpulmonary pressure changes with time, the transpulmonary pressure which corresponds to a part which is parallel to the horizontal axis on the waveform at the end of one respiratory cycle is the transpulmonary pressure at expiratory end of this respiratory cycle.

For example, as shown in FIG.6, the current transpulmonary pressure at expiratory end PtpE is 15cmH2O.

In step 3210, whether the transpulmonary pressure at expiratory end is lower than a second threshold, is determined. If it is lower than the second threshold, step 3220 is executed, otherwise, step 3200 is executed in the next respiratory cycle.

The second threshold is a preset parameter. For example, it can be a default empirical value in the program. This empirical value can be arranged as 0 cmH2O depending on clinical medical research or can also be a value which is calculated by a formula according to other parameters in the program. In some embodiments, the second threshold may also allow the user to adjust according to the actual situation of the patient.

In step 3220, the positive expiratory end pressure setting is guided to be increased. When guiding the positive expiratory end pressure to increase, the user can be guided by setting instructions. The guiding process through the setting instructions is similar as that for guiding the inspiratory pressure setting or inspiratory tidal volume setting to decrease, and is not repeated here.

Similarly, the user can also be guided by directly changing the original settings, and the guiding process is similar to the directly changing the original settings to guide the inspiratory pressure or inspiratory tidal volume to decrease, and is not repeated here.

This example can effectively reduce the risk of alveolar collapse by monitoring the transpulmonary pressure at expiratory end and guiding the end expiratory positive pressure setting to increase.

In some embodiments, it is also possible to guide the ventilation parameter setting according to the transpulmonary pressure at expiratory end and the transpulmonary pressure at inspiratory end at the same time, that is, to perform steps 3100 to 3120 and 3200 to 3220 respectively in each respiratory cycle, so as to guide the ventilation parameter setting at the end of the inspiratory phase and at the end of the expiratory phase.

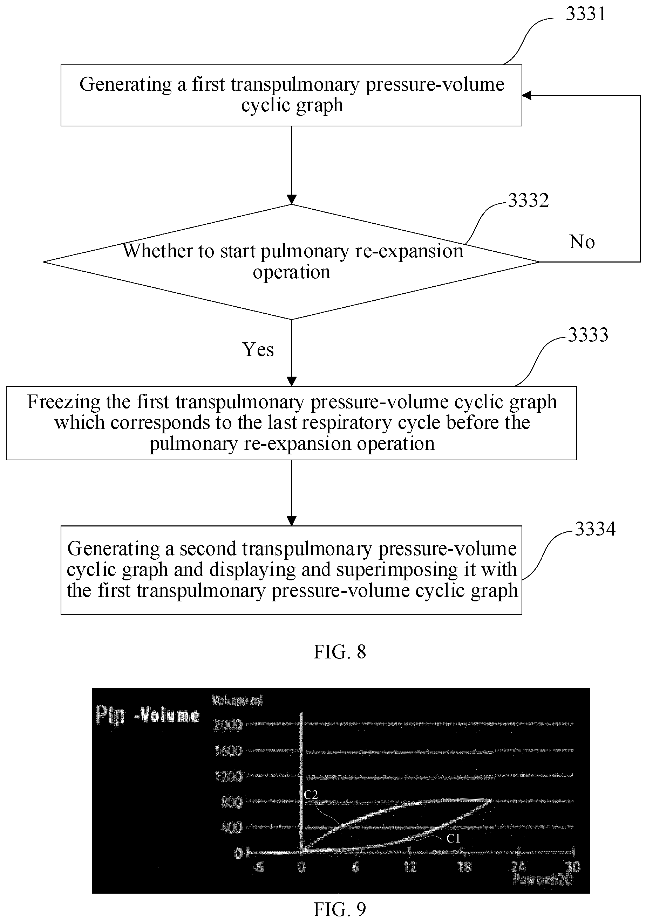

To facilitate the monitoring of the ventilation state of the patient, the processor also obtains the parameter waveform and/or parameter monitoring value according to the airway pressure value and/or esophageal pressure value, and outputs the parameter waveform and/or parameter monitoring value to the display interface for display, as shown in FIG. 6. The parameter waveform includes at least one of waveforms of airway pressure Paw and esophageal pressure (unshown), transpulmonary pressure Ptp. The parameter monitoring value includes at least one of transpulmonary pressure at inspiratory end PtpI, transpulmonary pressure at expiratory end PtpE, and transpulmonary pressure Ptp. In a preferred embodiment, the processor 14 also acquires the monitored pulmonary volume and generates a pressure-volume cyclic graph that changes with the respiratory cycle. The pressure-volume cyclic graph includes an esophageal pressure-volume cyclic graph and/or a transpulmonary pressure-volume cyclic graph. For example, during the ventilation monitoring of the patient, the generated transpulmonary pressure-volume cyclic graph is displayed on the display interface, as shown in FIG. 9. The pressure-volume cyclic graph is updated with the respiratory cycle, and the user can view the current risk of atelectasis according to the pressure-volume cyclic graph.

In another embodiment, as shown in FIG. 7, a flow diagram for guiding pulmonary re-expansion operation according to transpulmonary pressure of an embodiment includes following steps.

In step 3300, the transpulmonary pressure at expiratory end in a current respiratory cycle is obtained according to the monitored transpulmonary pressure. This step is similar as that of step 3200.

In step 3310, whether the transpulmonary pressure at expiratory end is lower than a second threshold (such as 0, or 0.5cmH2O), is determined. If it is lower than the second threshold, step 3320 is executed, otherwise, step 3300 is executed in the next respiratory cycle.

In step 3320, the pulmonary re-expansion operation is guided. The display interface of the display can instruct patterns, characters or other instruction information for pulmonary re-expansion. The processor can also directly change the ventilation parameters to increase the pressure which is applied to the alveolus for pulmonary re-expansion.

Pulmonary re-expansion is a process that is planned and induced to temporarily increase transpulmonary pressure. It can be understood that, by temporarily changing the ventilation parameters, the transpulmonary pressure is increased, so that the insufficiently ventilated or non-ventilated alveolus re-opens in a short time.

Of course, in step 3310, when determining whether to enter the pulmonary re-expansion operation, in addition to determining whether the obtained transpulmonary pressure at expiratory end is lower than the second threshold, it may also be necessary to conduct a comprehensive evaluation in combination with the duration time in which the transpulmonary pressure at expiratory end is lower than the second threshold, and one or more biological parameter information, such as blood oxygen and oxygenation and the likes.

At present, there are two common methods of pulmonary re-expansion, that is PEEP incremental or PEEP decremental method, and sustained inflation method.

First, the PEEP incremental method is taken as an example. PEEP incremental method means that when the patient is ventilated, the anesthesiologist increases the set values of the positive expiratory end pressure PEEP one cycle by one cycle to enable the alveolus to continuously increase the expiration-end volume, and then decreases the set values of positive expiratory end pressure PEEP one cycle by one cycle until reaching an appropriate PEEP value. The change point of the positive expiratory end pressure PEEP from increasing to decreasing is set by the user. The user determines the maximum PEEP that each patient can tolerate. Finding the best PEEP setting value through the pulmonary re-expansion operation of PEEP incremental or decremental method, can ensure that the alveolus is continuously open and never collapse. The appropriate positive expiratory end pressure is usually determined in the following way. If the transpulmonary pressure at expiratory end decreases from a positive value to being lower than or equal to the second threshold (e.g., zero) during the gradual decrease of the positive expiratory end pressure, then the positive expiratory end pressure in the previous cycle is the appropriate positive expiratory end pressure.

Preferably, in the step 3320 of implementing pulmonary re-expansion using the positive expiratory end pressure PEEP incremental or PEEP decremental method, the transpulmonary pressure at expiratory end during the pulmonary re-expansion operation is obtained in real time. During the gradual decrease of PEEP, if the transpulmonary pressure at expiratory end changes from a positive value to a value which is lower than the second threshold (e.g., 0) or even a negative value, the processor stops the pulmonary re-expansion operation. At the same time, the processor can also obtain the best PEEP setting value according to the pulmonary re-expansion results to guide the positive expiratory end pressure setting. For example, an alarm or instruction message pops up to inform the anesthesiologist that the current PEEP setting value may cause alveolar collapse and instruct the best PEEP value. Alternatively, the pulmonary re-expansion operation can be instructed to stop or automatically stopped, or an alarm instruction message pops up and PEEP is automatically arranged to a level slightly higher than the PEEP value before exiting the pulmonary re-expansion.

Sustained inflation method means that when the patient is ventilated, the user increases the airway pressure to a preset pressure value (Pressure Hold), and maintains this pressure value to continuous ventilate the patient for a period of time. The ventilation parameters of the sustained inflation method are set according to the conditions of different patients. For example, according to the experience of doctor and clinical research results, the ventilation parameters of normal adults are usually set as an airway pressure of 30cmH2O which lasts for 30 seconds.

In a specific embodiment, the processor 14 obtains the airway pressure value, esophageal pressure value and pulmonary volume in real time, obtains one or more of the parameter waveform, parameter trend graph, or parameter monitoring values according to the airway pressure value and or esophageal pressure value, and outputs one or more of the parameter waveform, parameter trend graph, or parameter monitoring values to the display interface for display. The parameter monitoring values include the transpulmonary pressure at inspiratory end PtpI, the transpulmonary pressure at expiratory end PtpE and/or transpulmonary pressure Ptp. The processor 14 generates the transpulmonary pressure-volume cyclic graph which changes with the respiratory cycle according to the transpulmonary pressure and pulmonary volume. For example, in the process of monitoring the ventilation of the patient, the generated transpulmonary pressure-volume cyclic graph is displayed on the display interface, and the transpulmonary pressure-volume cyclic graph is updated with the respiratory cycle. The user can view the current risk of atelectasis according to the transpulmonary pressure-volume cyclic graph. During pulmonary re-expansion operation, the trend of the transpulmonary pressure waveform, transpulmonary pressure-volume cyclic graph and/or the transpulmonary pressure at expiratory end PtpE are shown. In additional, the transpulmonary pressure-volume cyclic graphs before and after the pulmonary re-expansion, can also be shown to evaluate the effect of pulmonary re-expansion. The following is an example of the transpulmonary pressure-volume cyclic graph. The processing flow is shown in FIG. 8, which includes the following steps. In step 3331, the first transpulmonary pressure-volume cyclic graph is generated according to the monitored pulmonary volume and the monitored transpulmonary pressure.

The pulmonary volume can be calculated from the gas flow which is measured by the flow sensor in the respiratory line.

As shown in FIG.9, the first transpulmonary pressure-volume cyclic graph is generated according to the transpulmonary pressure and the pulmonary volume in one respiratory cycle, in which the transpulmonary pressure is the horizontal axis and the pulmonary volume is the vertical axis. When the patient is in the inspiratory phase, the pulmonary volume increases with the increase of transpulmonary pressure. Curve C1 in the figure is the transpulmonary pressure-volume curve in the inspiratory phase. When the patient is in the expiratory phase, the pulmonary volume decreases with the decrease of transpulmonary pressure. Curve C2 in the figure is the transpulmonary pressure volume curve in the expiratory phase. The two ends of the two curves overlap to form a closed loop.

In step 3332, whether to start the pulmonary re-expansion operation is determined. If the pulmonary re-expansion operation is determined to start, step 3333 is executed. If the pulmonary re-expansion operation is determined not to start, step 3331 is executed.

When the doctor needs to perform the pulmonary re-expansion operation, the pulmonary re-expansion operation instruction is triggered. The pulmonary re-expansion operation instruction can be triggered by a corresponding icon or menu option on the display interface, or by a physical switch. When the processor receives the pulmonary re-expansion operation instruction, it determines to start the pulmonary re-expansion operation.

In step 3333, a first transpulmonary pressure-volume cyclic graph which corresponds to the last respiratory cycle before the pulmonary re-expansion operation is frozen.

"Freeze" means that the image frame which includes the first transpulmonary pressure-volume cyclic graph is always displayed on the display interface.

In step 3334, a second transpulmonary pressure-volume cyclic graph is generated according to the monitored pulmonary volume and the monitored transpulmonary pressure.

It can be seen from the above that the first transpulmonary pressure-volume cyclic graph is the transpulmonary pressure-volume cyclic graph before the pulmonary re-expansion operation, and the second transpulmonary pressure-volume cyclic graph is the transpulmonary pressure-volume cyclic graph after the pulmonary re-expansion operation.

In step 3335, the second transpulmonary pressure-volume cyclic graph is displayed in real time. The second transpulmonary pressure-volume cyclic graph is outputted to the display interface of the display and displayed on a coordinate system which is next to that of the first transpulmonary pressure-volume cyclic graph or superimposed and displayed with the first transpulmonary pressure-volume cyclic graph, that is, in the same coordinate system of the first transpulmonary pressure-volume cyclic graph. The second transpulmonary pressure-volume cyclic graph is updated with the respiratory cycle.

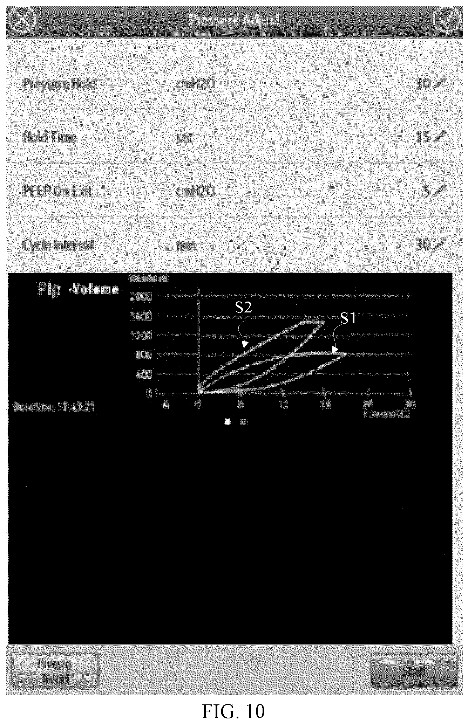

The two transpulmonary pressure-volume cyclic graphs are displayed in real time, and then users (such as doctors) can intuitively determine the effect of pulmonary re-expansion. For example, FIG. 10 is a schematic diagram showing a first transpulmonary pressure-volume cyclic graph and a second transpulmonary pressure-volume cyclic graph, which are superimposed and displayed on a display interface in an embodiment; wherein the first transpulmonary pressure-volume cyclic graph and the second transpulmonary pressure-volume cyclic graph are obtained before and after pulmonary re-expansion operation which is implemented through a sustained inflation method. The patient is ventilated with a positive expiratory end pressure of 5 cmH2O. The airway pressure is increased to a set pressure of 30 cmH2O every 30 minutes and lasted for 15s. S1 in the figure is first transpulmonary pressure-volume cyclic graph, and S2 in the figure is second transpulmonary pressure-volume cyclic graph. The greater the rotation deviation of S2 relative to S1, the better the effect of sustained inflation method.

In another embodiment, the transpulmonary pressure-volume cyclic graph can be replaced by, a waveform of the transpulmonary pressure at expiratory end which changes with time and the change trend of the waveform, to evaluate the effect of pulmonary re-expansion. Firstly, the waveform of the transpulmonary pressure at expiratory end, which changes with time, can be generated.

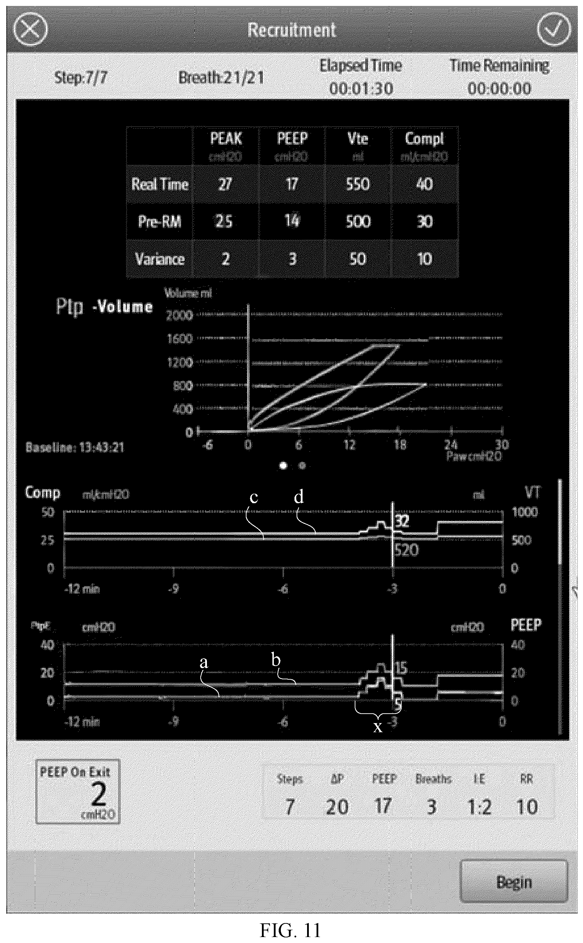

For example, as shown in FIG.11, curve a is the waveform of positive expiratory end pressure PEEP, curve b is the waveform of the current transpulmonary pressure at expiratory end PtpE, curve b is the waveform of tidal volume VT, and curve f is the waveform of pulmonary compliance.

In this example, the expiratory frequency RR is 10 times per minute, the initial positive expiratory end pressure is 2cmH2O, and the patient is ventilated with the initial positive expiratory end pressure within the time period which corresponds to the interval which is on the left side of interval x. In the time period which corresponds to the interval x shown in FIG.11, the patient is subjected to pulmonary re-expansion which is implemented through PEEP incremental or PEEP decremental method, in which the positive expiratory end pressure is increased by 3cmH2O every 6 seconds, and then decreased by 3cmH2O every 6 seconds after three times of increasing. After decreasing the positive expiratory end pressure for the third time, the vertical coordinate of curve b drops to zero, then the positive expiratory end pressure is increased by 3cmH2O in the next cycle, and the patient is ventilated with the positive expiratory end pressure, which is displayed in the form of visual parameters in FIG.11.

From the table in FIG.11, it can be seen visually that the real-time peak pressure (Real time) is 27cmH2O and the positive expiratory end pressure is 17cmH2O, which is 3cmH2O higher than the previous respiratory exercise (Pre-RM), while the current tidal volume is 550ml and the compliance is 40ml/cmH2O.

After obtaining the waveform of the transpulmonary pressure at expiratory end which changes with time, the pulmonary re-expansion effect can be evaluated according to the change trend of the transpulmonary pressure at expiratory end. This change trend can be characterized by the changed value T from the transpulmonary pressure at expiratory end on the right side of time interval x minus the transpulmonary pressure at expiratory end on the left side of time interval X. The greater the value of T, the better the effect of pulmonary re-expansion.

The ventilation monitoring device and method can monitor the transpulmonary pressure, and guide various ventilation parameters and pulmonary re-expansion operation and pulmonary re-expansion evaluation, according to the transpulmonary pressure, thus safely and effectively implementing the mechanical ventilation in clinical surgery.

Various exemplary embodiments are described herein. However, those skilled in the art recognizes that changes and modifications may be made to the exemplary embodiments without departing from the scope of this disclosure. For example, the various operation steps and the components used to perform the operation steps can be implemented in different ways according to the specific application or considering any number of cost functions associated with the operation of the system (for example, one or more steps can be deleted, modified or combined into other steps).

In addition, as understood by those skilled in the art, the principles herein can be reflected in a computer program product on a computer-readable storage medium which is pre-loaded with computer-readable program code. Any tangible, non-transitory computer-readable storage medium can be used, including magnetic storage devices (hard disk, floppy disk, etc.), optical storage devices (CD-ROM, DVD, Blu ray disk, etc.), flash memory and/or the likes. Computer program instructions can be loaded onto a general-purpose computer, a special-purpose computer, or other programmable data processing device to form a machine, so that these instructions executed on a computer or other programmable data processing device can generate a device to realize a specified function. These computer program instructions may also be stored in a computer-readable memory, which may instruct a computer or other programmable data processing device to operate in a specific manner, so that the instructions stored in the computer-readable memory can form a manufactured article, including an implementation device for realizing a specified function. Computer program instructions can also be loaded on a computer or other programmable data processing device, so that a series of operation steps are performed on the computer or other programmable device to generate a computer implemented process, so that the instructions executed on the computer or other programmable device can provide steps for realizing the specified functions.

Although the principles herein have been shown in various embodiments, many modifications of structures, arrangements, proportions, elements, materials, and components that are particularly applicable to specific environmental and operational requirements may be used without departing from the principles and scope of the present disclosure. The above modifications and other changes or amendments are included in the scope of this disclosure.

The foregoing specific description has been described with reference to various embodiments. However, those skilled in the art can recognize that various amendments and changes may be made without departing from the scope of this disclosure. Accordingly, consideration of this disclosure is illustrative rather than restrictive, and all such modifications are included within its scope. Similarly, there are solutions to the advantages, other advantages, and problems of the various embodiments as described above. However, the benefits, advantages, solutions to problems and any solution that can produce these elements or make them more explicit should not be interpreted as critical, necessary or necessary. The term "include" and any other variations thereof as used herein are non-exclusive inclusions, so that a process, method, article or device that includes a list of elements includes not only these elements, but also other elements that are not explicitly listed or do not belong to the process, method, system, article or device. In addition, the term "connection" and any other variation thereof as used herein refer to physical connection, electrical connection, magnetic connection, optical connection, communication connection, functional connection and/or any other connection.

Those skilled in the art recognize that many changes can be made to the details of the above embodiments without departing from the basic principles of this disclosure. Therefore, the scope of this disclosure shall be determined according to the following attached claims.

## Claims

1. A patient ventilation monitoring apparatus, comprising:
a sensor interface (10), which is configured to receive an airway pressure value of the patient, which is monitored by a first pressure sensor (19a), and an esophageal pressure value of the patient, which is monitored by a second pressure sensor (19d);
a processor (14), which is in signal connection with the sensor interface (10) and configured to receive the airway pressure value and the esophageal pressure value respectively, to obtain a transpulmonary pressure through calculating a difference between the airway pressure value and the esophageal pressure value, and to guide mechanical ventilation according to the transpulmonary pressure.

2. The patient ventilation monitoring apparatus according to claim 1, **characterized in that**, in order to guide mechanical ventilation according to the transpulmonary pressure, the processor (14) is specifically configured to:
obtain a transpulmonary pressure at inspiratory end and/or a transpulmonary pressure at expiratory end according to the transpulmonary pressure, and
guide ventilation parameter setting according to the transpulmonary pressure at inspiratory end and/or the transpulmonary pressure at expiratory end.

3. The patient ventilation monitoring apparatus according to claim 2, **characterized in that**, in order to guide ventilation parameter setting according to the transpulmonary pressure at inspiratory end and/or the transpulmonary pressure at expiratory end, the processor (14) is specifically configured to:
guide inspiratory pressure setting or tidal volume setting according to the transpulmonary pressure at inspiratory end; and/or guide positive expiratory end pressure setting according to the transpulmonary pressure at expiratory end.

4. The patient ventilation monitoring apparatus according to claim 3, **characterized in that**,
in order to guide inspiratory pressure setting or tidal volume setting according to the transpulmonary pressure at inspiratory end, the processor (14) is specifically configured to: guide to decrease the inspiratory pressure setting or the tidal volume setting when the transpulmonary pressure at inspiratory end is greater than a first threshold; and/or
in order to guide positive expiratory end pressure setting according to the transpulmonary pressure at expiratory end, the processor (14) is specifically configured to: guide to increase the positive expiratory end pressure setting, when the transpulmonary pressure at expiratory end is lower than a second threshold.

5. The patient ventilation monitoring apparatus according to claim 2, **characterized in that**, in order to guide ventilation parameter setting according to the transpulmonary pressure at inspiratory end and/or the transpulmonary pressure at expiratory end, the processor (14) is specifically configured to:
guide the ventilation parameter setting according to the transpulmonary pressure at inspiratory end and/or the transpulmonary pressure at expiratory end; or
set a ventilation parameter according to the transpulmonary pressure at inspiratory end and/or the transpulmonary pressure at expiratory end.

6. The patient ventilation monitoring apparatus according to claim 1, **characterized in that**, in order to guide mechanical ventilation according to the transpulmonary pressure, the processor (14) is specifically configured to:
obtain a transpulmonary pressure at expiratory end according to the transpulmonary pressure, and
implement pulmonary re-expansion operation according to the transpulmonary pressure at expiratory end.

7. The patient ventilation monitoring apparatus according to claim 6, **characterized in that**,
in order to implement pulmonary re-expansion operation according to the transpulmonary pressure at expiratory end, the processor (14) is specifically configured to: guide to implement the pulmonary re-expansion operation or directly implement the pulmonary re-expansion operation, when the transpulmonary pressure at expiratory end is lower than a second threshold; and/or
during implementing the pulmonary re-expansion operation through a positive expiratory end pressure incremental or decremental method, the processor (14) is further configured to: stop the pulmonary re-expansion operation, when detecting that the transpulmonary pressure at expiratory end is lower than a second threshold during a gradual decrease of the positive expiratory end pressure.

8. The patient ventilation monitoring apparatus according to claim 1, **characterized in that**, the processor (14) is further configured to acquire one or more of parameter waveform, parameter trend diagram or parameter monitoring value and send the one or more of parameter waveform, parameter trend diagram or parameter monitoring value to a display interface for display.

9. The patient ventilation monitoring apparatus according to claim 8, **characterized in that**, the parameter waveform comprises at least one of airway pressure waveform, esophageal pressure waveform and transpulmonary pressure waveform that change with time; the parameter trend diagram comprises at least one of airway pressure trend diagram, esophageal pressure trend diagram and transpulmonary pressure trend diagram; and the parameter monitoring value comprises at least one of transpulmonary pressure at inspiratory end, transpulmonary pressure at expiratory end and the transpulmonary pressure.

10. The patient ventilation monitoring apparatus according to claim 1, **characterized in that**,
the processor (14) is further configured to acquire a pulmonary volume and to generate a pressure-volume cyclic graph that changes with a respiratory cycle;
wherein the pressure-volume cyclic graph comprises an esophageal pressure-volume cyclic graph and/or a transpulmonary pressure-volume cyclic graph; and/or
the processor (14) is further configured to output a first transpulmonary pressure-volume cyclic graph and a second transpulmonary pressure-volume cyclic graph to a display interface of a display for superimposed display after detecting that pulmonary re-expansion operation is started; wherein the first transpulmonary pressure-volume cyclic graph is a transpulmonary pressure-volume cyclic graph before the pulmonary re-expansion operation, and the second transpulmonary pressure-volume cyclic graph is a transpulmonary pressure-volume cyclic graph after the pulmonary re-expansion operation.

11. An anesthetic ventilation device, comprising:
a gas source interface (10) , which is connected with an external gas source;
a respiratory line (13), which is configured to connect the gas source interface (10) with a respiratory system of patient, to input gas, which is provided by the gas source, to the patient, and to receive exhaled gas of the patient;
an anesthetic drug output apparatus (12), which is configured to mix stored anesthetic drug with input gas, and to output the gas which is mixed with the anesthetic drug to the respiratory line (13);
a respiratory assistance apparatus (11), which is configured to provide respiratory support power for controlling the gas, which is provided by the gas source, and the gas, which is mixed with the anesthetic drug and outputted by the anesthetic drug output apparatus (12), to be outputted to the patient; or for recycling the exhaled gas of the patient; or for discharging the exhaled gas of the patient to external environment; wherein the respiratory assistance apparatus (11) comprises a computer-controlled ventilation module and/or a manual ventilation module; and
the patient ventilation monitoring apparatus according to any one of claims 1-10.

12. An apparatus for implementing patient ventilation monitoring on an anesthetic ventilation device, comprising:
a memory, which is configured to store a program;
a processor, which is configured to implement a patient ventilation monitoring method, by executing the program which is stored in the memory;
the patient ventilation monitoring method comprises:
acquiring an airway pressure value and an esophageal pressure value of the patient;
obtaining a transpulmonary pressure through calculating a difference between the airway pressure value and the esophageal pressure value, and
guiding mechanical ventilation according to the transpulmonary pressure.

13. The apparatus for implementing patient ventilation monitoring on an anesthetic ventilation device according to claim 12, **characterized in that**, guiding mechanical ventilation according to the transpulmonary pressure, specifically comprises:
obtaining a transpulmonary pressure at inspiratory end and/or a transpulmonary pressure at expiratory end according to the transpulmonary pressure, and
guiding ventilation parameter setting according to the transpulmonary pressure at inspiratory end and/or the transpulmonary pressure at expiratory end;
wherein guiding ventilation parameter setting according to the transpulmonary pressure at inspiratory end and/or the transpulmonary pressure at expiratory end, specifically comprises: guiding inspiratory pressure setting or tidal volume setting according to the transpulmonary pressure at inspiratory end; and/or guiding positive expiratory end pressure setting according to the transpulmonary pressure at expiratory end; wherein guiding inspiratory pressure setting or tidal volume setting according to the transpulmonary pressure at inspiratory end, specifically comprises: guiding to decrease the inspiratory pressure setting or the tidal volume setting when the transpulmonary pressure at inspiratory end is greater than a first threshold; and/or guiding positive expiratory end pressure setting according to the transpulmonary pressure at expiratory end, specifically comprises: guiding to increase the positive expiratory end pressure setting, when the transpulmonary pressure at expiratory end is lower than a second threshold; and/or
wherein guiding ventilation parameter setting according to the transpulmonary pressure at inspiratory end and/or the transpulmonary pressure at expiratory end, specifically comprises: guiding the ventilation parameter setting according to the transpulmonary pressure at inspiratory end and/or the transpulmonary pressure at expiratory end; or setting a ventilation parameter according to the transpulmonary pressure at inspiratory end and/or the transpulmonary pressure at expiratory end.

14. The apparatus for implementing patient ventilation monitoring on an anesthetic ventilation device according to claim 12, **characterized in that**, guiding mechanical ventilation according to a monitored transpulmonary pressure, specifically comprises: obtaining a transpulmonary pressure at expiratory end according to the monitored transpulmonary pressure, and implementing pulmonary re-expansion operation according to the transpulmonary pressure at expiratory end;
wherein implementing pulmonary re-expansion operation according to the transpulmonary pressure at expiratory end, specifically comprises: guiding to implement the pulmonary re-expansion operation or directly implement the pulmonary re-expansion operation, when the transpulmonary pressure at expiratory end is lower than a second threshold; or during implementing the pulmonary re-expansion operation through a positive expiratory end pressure incremental or decremental method; stopping the pulmonary re-expansion operation, when detecting that the transpulmonary pressure at expiratory end is lower than a second threshold during a gradual decrease of the positive expiratory end pressure;
wherein the method further comprises guiding positive expiratory end pressure setting according to the transpulmonary pressure at expiratory end after implementing the pulmonary re-expansion operation.

15. The apparatus for implementing patient ventilation monitoring on an anesthetic ventilation device according to claim 12, **characterized in that**, the method further comprises:
acquiring one or more of parameter waveform, parameter trend diagram or parameter monitoring value; and sending the one or more of parameter waveform, parameter trend diagram or parameter monitoring value to a display interface for display;
acquiring a pulmonary volume and generating a pressure-volume cyclic graph that changes with a respiratory cycle; wherein the pressure-volume cyclic graph comprises an esophageal pressure-volume cyclic graph and/or a transpulmonary pressure-volume cyclic graph; or
outputting a first transpulmonary pressure-volume cyclic graph and a second transpulmonary pressure-volume cyclic graph to a display interface of a display for superimposed display after detecting that pulmonary re-expansion operation is started; wherein the first transpulmonary pressure-volume cyclic graph is a transpulmonary pressure-volume cyclic graph before the pulmonary re-expansion operation, and the second transpulmonary pressure-volume cyclic graph is a transpulmonary pressure-volume cyclic graph after the pulmonary re-expansion operation.

## Patentansprüche

1. Ein Gerät zur Überwachung der Beatmung eines Patienten, das Folgendes umfasst:
eine Sensorschnittstelle (10) zur Erfassung eines Atemwegsdruckwerts des Patienten, der über einen ersten Drucksensor (19a) überwacht wird, und eines Ösophagusdruckwerts des Patienten, der über einen zweiten Drucksensor (19d) überwacht wird;
einen Prozessor (14), der mit der Sensorschnittstelle (10) über ein Signal verbunden ist und so ausgelegt ist, dass er den Atemwegsdruckwert bzw. den Ösophagusdruckwert erfasst, um einen transpulmonalen Druck durch Berechnung der Differenz zwischen dem Atemwegsdruckwert und dem Ösophagusdruckwert zu erhalten und die mechanische Beatmung entsprechend des transpulmonalen Drucks anzuleiten.

2. Gerät zur Überwachung der Beatmung eines Patienten nach Anspruch 1, mit der Eigenschaft, dass der Prozessor (14), um die mechanische Beatmung entsprechend des transpulmonalen Drucks anzuleiten, insbesondere so ausgelegt ist,
dass er einen transpulmonalen Druck am Ende der Inspiration und/oder am Ende der Exspiration entsprechend des transpulmonalen Drucks ermittelt
und die Einstellung der Beatmungsparameter entsprechend des transpulmonalen Drucks am Ende der Inspiration und/oder am Ende der Exspiration anleitet.

3. Gerät zur Überwachung der Beatmung eines Patienten nach Anspruch 2, mit der Eigenschaft, dass der Prozessor (14), um die Einstellung der Beatmungsparameter entsprechend des transpulmonalen Drucks am Ende der Inspiration und/oder der Exspiration anzuleiten, insbesondere so ausgelegt ist,
dass er die Einstellung des Inspirationsdrucks oder des Tidalvolumens entsprechend des transpulmonalen Drucks am Ende der Inspiration anleitet; und/oder die Einstellung des positiven endexspiratorischen Drucks (PEEP) entsprechend des transpulmonalen Drucks am Ende der Exspiration anleitet.

4. Gerät zur Überwachung der Beatmung eines Patienten nach Anspruch 3, mit der Eigenschaft,
dass der Prozessor (14), um die Einstellung des Inspirationsdrucks oder des Tidalvolumens entsprechend des transpulmonalen Drucks am Ende der Inspiration anzuleiten, insbesondere so ausgelegt ist, dass er: die Senkung der Einstellung des Inspirationsdrucks oder des Tidalvolumens anleitet, wenn der transpulmonale Druck am Ende der Inspiration größer als ein erster Schwellenwert ist; und/oder
um die Einstellung des positiven endexspiratorischen Drucks (PEEP) entsprechend des transpulmonalen Drucks am Ende der Exspiration anzuleiten, ist der Prozessor (14) insbesondere so ausgelegt, dass er eine Erhöhung der Einstellung des positiven endexspiratorischen Drucks anleitet, wenn der transpulmonale Druck am Ende der Exspiration niedriger als ein zweiter Schwellenwert ist.

5. Gerät zur Überwachung der Beatmung eines Patienten nach Anspruch 2, mit der Eigenschaft, dass der Prozessor (14), um die Einstellung der Beatmungsparameter entsprechend des transpulmonalen Drucks am Ende der Inspiration und/oder
der Exspiration anzuleiten, insbesondere so ausgelegt ist, dass er: die Einstellung der Beatmungsparameter entsprechend des transpulmonalen Drucks am Ende der Inspiration und/oder der Exspiration anleitet;
oder einen Beatmungsparameter entsprechend des transpulmonalen Drucks am Ende der Inspiration und/oder der Exspiration einstellt.

6. Gerät zur Überwachung der Beatmung eines Patienten nach Anspruch 1, mit der Eigenschaft, dass der Prozessor (14), um die mechanische Beatmung entsprechend des transpulmonalen Drucks anzuleiten, insbesondere so ausgelegt ist, dass er einen transpulmonalen Druck am Ende der Exspiration entsprechend des transpulmonalen Drucks ermittelt und eine Lungenreexpansion entsprechend des transpulmonalen Drucks am Ende der Exspiration durchführt.

7. Gerät zur Überwachung der Beatmung eines Patienten nach Anspruch 6, mit der Eigenschaft,
dass der Prozessor (14), um eine Lungenreexpansion entsprechend des transpulmonalen Drucks am Ende der Exspiration durchzuführen, insbesondere so ausgelegt ist, dass er die Durchführung der Lungenreexpansion anleitet oder direkt durchführt, wenn der transpulmonale Druck am Ende der Exspiration niedriger als ein zweiter Schwellenwert ist; und/oder
während der Durchführung der Lungenreexpansion mittels eines Verfahrens mit schrittweiser Erhöhung oder Senkung des positiven endexspiratorischen Drucks ist der Prozessor (14) ferner so konfiguriert, dass er die Lungenreexpansion stoppt, wenn während einer allmählichen Senkung des positiven endexspiratorischen Drucks festgestellt wird, dass der transpulmonale Druck am Ende der Exspiration unterhalb eines zweiten Schwellenwerts liegt.

8. Gerät zur Überwachung der Beatmung eines Patienten nach Anspruch 1, mit der Eigenschaft, dass der Prozessor (14) ferner so ausgelegt ist, dass er einen oder mehrere der folgenden Parameter erfasst: Parameterkurve, Parametertrenddiagramm oder Parameterüberwachungswert, und den/die erfassten Parameter an eine Anzeigeschnittstelle zur Anzeige übermittelt

9. Gerät zur Überwachung der Beatmung eines Patienten nach Anspruch 8, mit der Eigenschaft, dass die Parameterkurve mindestens entweder eine Atemwegsdruckkurve, eine Ösophagusdruckkurve oder eine transpulmonale Druckkurve umfasst, die jeweils in Abhängigkeit von der Zeit stehen; das Parametertrenddiagramm umfasst mindestens entweder ein Atemwegsdrucktrenddiagramm, ein Ösophagusdrucktrenddiagramm oder ein transpulmonales Drucktrenddiagramm; und der Parameterüberwachungswert umfasst mindestens einen transpulmonalen Druck am Ende der Inspiration, einen transpulmonalen Druck am Ende der Exspiration oder einen transpulmonalen Druck.

10. Gerät zur Überwachung der Beatmung eines Patienten nach Anspruch 1, mit der Eigenschaft,
dass der Prozessor (14) ferner so konfiguriert ist, dass er ein Lungenvolumen erfasst und ein Druck-Volumen-Zyklusdiagramm erzeugt, das sich mit dem Atemzyklus ändert; wobei das Druck-Volumen-Zyklusdiagramm ein Ösophagusdruck-Volumen-Zyklusdiagramm und/oder
ein transpulmonales Druck-Volumen-Zyklusdiagramm umfasst; und/oder der Prozessor (14) ferner so ausgelegt ist, dass er ein erstes und ein zweites transpulmonales Druck-Volumen-Zyklusdiagramm an eine Anzeigeschnittstelle eines Displays zur überlagerten Darstellung überträgt, nachdem festgestellt wurde, dass eine Lungenreexpansion eingeleitet wurde; wobei das erste transpulmonale Druck-Volumen-Zyklusdiagramm dem Diagramm vor der Lungenreexpansion und das zweite Diagramm das Diagramm nach der Lungenreexpansion entspricht;

11. Ein Anästhesiebeatmungsgerät, das Folgendes umfasst:
eine Gasquellenschnittstelle (10), die mit einer externen Gasquelle verbunden ist;
eine Atemleitung (13), die die Gasquellenschnittstelle (10) mit einem Atmungssystem des Patienten verbindet, um Gas, das von der Gasquelle bereitgestellt wird, dem Patienten zuzuführen und ausgeatmetes Gas des Patienten aufzunehmen;
eine Narkosemittel-Abgabevorrichtung (12), die gespeichertes Narkosemittel mit dem zugeführten Gas mischt und an die Atemleitung (13) abgibt;
ein Beatmungsgerät (11), das als Atemhilfe das von der Gasquelle bereitgestellte Gas sowie das mit dem Narkosemittel vermischte und von der Narkosemittel-Abgabevorrichtung (12) ausgegebene Gas zum Patienten leitet; oder das ausgeatmete Gas des Patienten zurückführt oder an die Außenumgebung abführt, wobei das Beatmungsgerät(11) ein computergesteuertes Beatmungsmodul und/oder ein manuelles Beatmungsmodul beinhaltet;
das Beatmungsüberwachungsgerät gemäß einem der Ansprüche 1 bis 10.

12. Eine Vorrichtung zur Durchführung der Überwachung der Patientenbeatmung an einem Anästhesiebeatmungsgerät, die Folgendes umfasst:
einen Speicher zur Speicherung eines Programms;
einen Prozessor, der das gespeicherte Programm zur Durchführung der Überwachung der Patientenbeatmung implementiert;
wobei das Verfahren zur Überwachung der Patientenbeatmung Folgendes umfasst:
Erfassen eines Atemwegsdruckwerts und eines Ösophagusdruckwerts des Patienten;
Ermitteln eines transpulmonalen Drucks durch Berechnen der Differenz zwischen dem Atemwegsdruckwert und dem Ösophagusdruckwert; und
Anleitung der mechanischen Beatmung entsprechend des transpulmonalen Drucks.

13. Die Vorrichtung zur Durchführung der Überwachung der Patientenbeatmung an einem Anästhesiebeatmungsgerät nach Anspruch 12, mir der Eigenschaft, dass die Anleitung der mechanischen Beatmung entsprechend des transpulmonalen Drucks Folgendes umfasst:
Ermitteln eines transpulmonalen Drucks am Ende der Inspiration und/oder am Ende der Exspiration entsprechend des transpulmonalen Drucks; und
Anleitung der Einstellung von Beatmungsparametern entsprechend des transpulmonalen Drucks am Ende der Inspiration und/oder am Ende der Exspiration;,
wobei die Anleitung der Einstellung von Beatmungsparametern entsprechend des transpulmonalen Drucks am Ende der Inspiration und/oder der Exspiration Folgendes umfasst: Anleitung zur Einstellung des Inspirationsdrucks oder des Tidalvolumens entsprechend des transpulmonalen Drucks am Ende der Inspiration; und/oder Anleitung zur Einstellung des positiven endexspiratorischen Drucks entsprechend des transpulmonalen Drucks am Ende der Exspiration; wobei die Anleitung zur Einstellung des Inspirationsdrucks oder des Tidalvolumens entsprechend des transpulmonalen Drucks am Ende der Inspiration Folgendes umfasst: Anleitung zur Senkung der Einstellung des Inspirationsdrucks oder des Tidalvolumens, wenn der transpulmonale Druck am Ende der Inspiration größer als ein erster Schwellenwert ist; und/oder
Anleitung zur Einstellung des positiven endexspiratorischen Drucks entsprechend des transpulmonalen Drucks am Ende der Exspiration, mit der Anleitung zur Erhöhung des positiven endexspiratorischen Drucks, wenn der transpulmonale Druck am Ende der Exspiration kleiner als ein zweiter Schwellenwert ist; und/oder wobei die Anleitung der Einstellung von Beatmungsparametern entsprechend des transpulmonalen Drucks am Ende der Inspiration und/oder am Ende der Exspiration insbesondere die Anleitung zur Einstellung von Beatmungsparametern entsprechend des transpulmonalen Drucks am Ende der Inspiration und/oder am Ende der Exspiration umfasst; oder Festlegen eines Beatmungsparameters entsprechend des transpulmonalen Drucks am Ende der Inspiration und/oder am Ende der Exspiration.

14. Die Vorrichtung zur Durchführung der Überwachung der Patientenbeatmung an einem Anästhesiebeatmungsgerät nach Anspruch 12, mit der Eigenschaft, dass die Anleitung der mechanischen Beatmung entsprechend eines überwachten transpulmonalen Drucks insbesondere Folgendes umfasst: Ermitteln eines transpulmonalen Drucks am Ende der Exspiration entsprechend des überwachten transpulmonalen Drucks und Durchführung einer Lungenreexpansion entsprechend des transpulmonalen Drucks am Ende der Exspiration;
wobei die Durchführung einer Lungenreexpansion entsprechend des transpulmonalen Drucks am Ende der Exspiration insbesondere Folgendes umfasst: Anleitung zur Durchführung der Lungenreexpansion oder direkte Durchführung der Lungenreexpansion, wenn der transpulmonale Druck am Ende der Exspiration unter einem zweiten Schwellenwert liegt; oder während der Durchführung der Lungenreexpansion durch eine Methode mit schrittweiser Erhöhung oder Senkung des positiven endexspiratorischen Drucks: Beenden der Lungenreexpansion, wenn festgestellt wird, dass der transpulmonale Druck am Ende der Exspiration während einer allmählichen Senkung des positiven endexspiratorischen Drucks unter einen zweiten Schwellenwert fällt;
wobei das Verfahren weiterhin die Anleitung zur Einstellung des positiven endexspiratorischen Drucks entsprechend des transpulmonalen Drucks am Ende der Exspiration nach Durchführung der Lungenreexpansion beinhaltet.

15. Die Vorrichtung zur Durchführung der Überwachung der Patientenbeatmung an einem Anästhesiebeatmungsgerät nach Anspruch 12, mit der Eigenschaft, dass das Verfahren weiterhin Folgendes umfasst:
Erfassen eines oder mehrerer der folgenden Elemente: Parameterwellenform, Parametertrendiagramm oder Parameterüberwachungswert; und Übermitteln der Parameterwellenform, des Parametertrendiagramms oder des Parameterüberwachungswerts an eine Anzeigeschnittstelle zur Darstellung;
Erfassen eines Lungenvolumens und Erzeugen eines Druck-Volumen-Zyklendiagramms, das sich mit einem Atemzyklus ändert; wobei das Druck-Volumen-Zyklendiagramm ein Ösophagusdruck-Volumen-Zyklendiagramm und/oder ein transpulmonales Druck-Volumen-Zyklendiagramm umfasst; oder
Ausgeben eines ersten transpulmonalen Druck-Volumen-Zyklendiagramms und eines zweiten transpulmonalen Druck-Volumen-Zyklendiagramms an eine Anzeigeoberfläche eines Displays zur überlagerten Anzeige nach Feststellung, dass eine Lungenreexpansion eingeleitet wurde; wobei das erste transpulmonale Druck-Volumen-Zyklendiagramm einem transpulmonalen Druck-Volumen-Zyklendiagramm vor der Lungenreexpansion und das zweite transpulmonale Druck-Volumen-Zyklendiagramm einem transpulmonalen Druck-Volumen-Zyklendiagramm nach der Lungenreexpansion entspricht.

## Revendications

1. Appareil de surveillance de ventilation de patient, comprenant :
une interface de capteur (10), laquelle est configurée pour recevoir une valeur de pression de voie aérienne du patient, laquelle est surveillée par un premier capteur de pression (19a), et une valeur de pression œsophagienne du patient, laquelle est surveillée par un second capteur de pression (19d) ;
un processeur (14), lequel est en liaison de signal avec l'interface de capteur (10) et configuré pour recevoir la valeur de pression de voie aérienne et la valeur de pression œsophagienne respectivement, pour obtenir une pression transpulmonaire en calculant une différence entre la valeur de pression de voie aérienne et la valeur de pression œsophagienne, et pour guider une ventilation mécanique selon la pression transpulmonaire.

2. Appareil de surveillance de ventilation de patient selon la revendication 1, **caractérisé en ce que**, pour guider une ventilation mécanique selon la pression transpulmonaire, le processeur (14) est en particulier configuré pour :
obtenir une pression transpulmonaire en fin d'inspiration et/ou une pression transpulmonaire en fin d'expiration selon la pression transpulmonaire, et
guider un réglage de paramètre de ventilation selon la pression transpulmonaire en fin d'inspiration et/ou la pression transpulmonaire en fin d'expiration.

3. Appareil de surveillance de ventilation de patient selon la revendication 2, **caractérisé en ce que**, pour guider un réglage de paramètre de ventilation selon la pression transpulmonaire en fin d'inspiration et/ou la pression transpulmonaire en fin d'expiration, le processeur (14) est en particulier configuré pour :
guider un réglage de pression inspiratoire ou un réglage de volume courant selon la pression transpulmonaire en fin d'inspiration ; et/ou guider un réglage de pression en fin d'expiration positive selon la pression transpulmonaire en fin d'expiration.

4. Appareil de surveillance de ventilation de patient selon la revendication 3, **caractérisé en ce que**,
pour guider un réglage de pression inspiratoire ou un réglage de volume courant selon la pression transpulmonaire en fin d'inspiration, le processeur (14) est en particulier configuré pour : guider de sorte à diminuer le réglage de pression inspiratoire ou le réglage de volume courant lorsque la pression transpulmonaire en fin d'inspiration est supérieure à un premier seuil ; et/ou
pour guider un réglage de pression en fin d'expiration positive selon la pression transpulmonaire en fin d'expiration, le processeur (14) est en particulier configuré pour : guider de sorte à augmenter le réglage de pression en fin d'expiration positive, lorsque la pression transpulmonaire en fin d'expiration est inférieure à un second seuil.

5. Appareil de surveillance de ventilation de patient selon la revendication 2, **caractérisé en ce que**, pour guider un réglage de paramètre de ventilation selon la pression transpulmonaire en fin d'inspiration et/ou la pression transpulmonaire en fin d'expiration, le processeur (14) est en particulier configuré pour :
guider le réglage de paramètre de ventilation selon la pression transpulmonaire en fin d'inspiration et/ou la pression transpulmonaire en fin d'expiration ; ou
régler un paramètre de ventilation selon la pression transpulmonaire en fin d'inspiration et/ou la pression transpulmonaire en fin d'expiration.

6. Appareil de surveillance de ventilation de patient selon la revendication 1, **caractérisé en ce que**, pour guider une ventilation mécanique selon la pression transpulmonaire, le processeur (14) est en particulier configuré pour :
obtenir une pression transpulmonaire en fin d'expiration selon la pression transpulmonaire, et
mettre en œuvre une opération de regonflement pulmonaire selon la pression transpulmonaire en fin d'expiration.

7. Appareil de surveillance de ventilation de patient selon la revendication 6, **caractérisé en ce que**,
pour mettre en œuvre une opération de regonflement pulmonaire selon la pression transpulmonaire en fin d'expiration, le processeur (14) est en particulier configuré pour : guider de sorte à mettre en œuvre l'opération de regonflement pulmonaire ou directement mettre en œuvre l'opération de regonflement pulmonaire, lorsque la pression transpulmonaire en fin d'expiration est inférieure à un second seuil ; et/ou
durant la mise en œuvre de l'opération de regonflement pulmonaire par un procédé incrémental ou décrémental de pression en fin d'expiration positive, le processeur (14) est en outre configuré pour : arrêter l'opération de regonflement pulmonaire s'il est détecté que la pression transpulmonaire en fin d'expiration est inférieure à un second seuil durant une diminution graduelle de la pression en fin d'expiration positive.

8. Appareil de surveillance de ventilation de patient selon la revendication 1, **caractérisé en ce que**, le processeur (14) est en outre configuré pour acquérir un ou plusieurs éléments parmi une forme d'onde de paramètre, un diagramme de tendance de paramètre ou une valeur de surveillance de paramètre et envoyer les un ou plusieurs éléments parmi une forme d'onde de paramètre, un diagramme de tendance de paramètre ou une valeur de surveillance de paramètre à une interface d'affichage pour affichage.

9. Appareil de surveillance de ventilation de patient selon la revendication 8, **caractérisé en ce que**, la forme d'onde de paramètre comprend au moins l'une d'une forme d'onde de pression de voie aérienne, d'une forme d'onde de pression œsophagienne et d'une forme d'onde de pression transpulmonaire qui varient dans le temps ; le diagramme de tendance de paramètre comprend au moins l'un d'un diagramme de tendance de pression de voie aérienne, d'un diagramme de tendance de pression œsophagienne et d'un diagramme de tendance de pression transpulmonaire ; et la valeur de surveillance de paramètre comprend au moins l'une d'une pression transpulmonaire en fin d'inspiration, d'une pression transpulmonaire en fin d'expiration et de la pression transpulmonaire.

10. Appareil de surveillance de ventilation de patient selon la revendication 1, **caractérisé en ce que**,
le processeur (14) est en outre configuré pour acquérir un volume pulmonaire et pour générer un graphique cyclique pression-volume qui varie avec un cycle respiratoire ; où le graphique cyclique pression-volume comprend un graphique cyclique pression œsophagienne-volume et/ou un graphique cyclique pression transpulmonaire-volume ; et/ou
le processeur (14) est en outre configuré pour fournir un premier graphique cyclique pression transpulmonaire-volume et un second graphique cyclique pression transpulmonaire-volume à une interface d'affichage d'un affichage pour un affichage superposé après détection qu'une opération de regonflement pulmonaire a commencé ; où le premier graphique cyclique pression transpulmonaire-volume est un graphique cyclique pression transpulmonaire-volume avant l'opération de regonflement pulmonaire, et le second graphique cyclique pression transpulmonaire-volume est un graphique cyclique pression transpulmonaire-volume après l'opération de regonflement pulmonaire.

11. Dispositif de ventilation anesthésique, comprenant :
une interface de source de gaz (10), laquelle est reliée à un source de gaz externe ;
une ligne respiratoire (13), laquelle est configurée pour relier l'interface de source de gaz (10) à un système respiratoire de patient, pour fournir du gaz, lequel est fourni par la source de gaz, au patient, et pour recevoir un gaz exhalé du patient ;
un appareil de fourniture de médicament anesthésique (12), lequel est configuré pour mélanger un médicament anesthésique stocké avec du gaz fourni, et pour fournir le gaz qui est mélangé avec le médicament anesthésique à la ligne respiratoire (13) ;
un appareil d'assistance respiratoire (11), lequel est configuré pour fournir une puissance d'aide respiratoire pour commander le gaz, qui est fourni par la source de gaz, et le gaz, qui est mélangé avec le médicament anesthésique et fourni par l'appareil de fourniture de médicament anesthésique (12), à fournir au patient ; ou pour recycler le gaz exhalé du patient ; ou pour évacuer le gaz exhalé du patient vers un environnement externe ; où l'appareil d'assistance respiratoire (11) comprend un module de ventilation commandé par ordinateur et/ou un module de ventilation manuel ; et
l'appareil de surveillance de ventilation de patient selon l'une quelconque des revendications 1 à 10.

12. Appareil destiné à mettre en œuvre une surveillance de ventilation de patient sur un dispositif de ventilation anesthésique, comprenant :
une mémoire, qui est configurée pour stocker un programme ;
un processeur, qui est configuré pour mettre en œuvre un procédé de surveillance de ventilation de patient, en exécutant le programme qui est mémorisé dans la mémoire ;
le procédé de surveillance de ventilation de patient comprend les étapes consistant à :
acquérir une valeur de pression de voie aérienne et une valeur de pression œsophagienne du patient ;
obtenir une pression transpulmonaire en calculant une différence entre la valeur de pression de voie aérienne et la valeur de pression œsophagienne, et
guider une ventilation mécanique selon la pression transpulmonaire.

13. Appareil destiné à mettre en œuvre une surveillance de ventilation de patient sur un dispositif de ventilation anesthésique selon la revendication 12, **caractérisé en ce que**, le fait de guider une ventilation mécanique selon la pression transpulmonaire comprend en particulier :
le fait d'obtenir une pression transpulmonaire en fin d'inspiration et/ou une pression transpulmonaire en fin d'expiration selon la pression transpulmonaire, et
guider un réglage de paramètre de ventilation selon la pression transpulmonaire en fin d'inspiration et/ou la pression transpulmonaire en fin d'expiration,
où le fait de guider un réglage de paramètre de ventilation selon la pression transpulmonaire en fin d'inspiration et/ou la pression transpulmonaire en fin d'expiration comprend en particulier : le fait de guider un réglage de pression inspiratoire ou un réglage de volume courant selon la pression transpulmonaire en fin d'inspiration ; et/ou guider un réglage de pression en fin d'expiration positive selon la pression transpulmonaire en fin d'expiration ; où le fait de guider un réglage de pression inspiratoire ou un réglage de volume courant selon la pression transpulmonaire en fin d'inspiration comprend en particulier : le fait de guider de sorte à diminuer le réglage de pression inspiratoire ou le réglage de volume courant lorsque la pression transpulmonaire en fin d'inspiration est supérieure à un premier seuil ; et/ou le fait de guider un réglage de pression en fin d'expiration positive selon la pression transpulmonaire en fin d'expiration comprend en particulier : le fait de guider de sorte à augmenter le réglage de pression en fin d'expiration positive, lorsque la pression transpulmonaire en fin d'expiration est inférieure à un second seuil ; et/ou
où le fait de guider un réglage de paramètre de ventilation selon la pression transpulmonaire en fin d'inspiration et/ou la pression transpulmonaire en fin d'expiration comprend en particulier : le fait de guider le réglage de paramètre de ventilation selon la pression transpulmonaire en fin d'inspiration et/ou la pression transpulmonaire en fin d'expiration ; ou le fait de régler un paramètre de ventilation selon la pression transpulmonaire en fin d'inspiration et/ou la pression transpulmonaire en fin d'expiration.

14. Appareil destiné à mettre en œuvre une surveillance de ventilation de patient sur un dispositif de ventilation anesthésique selon la revendication 12, **caractérisé en ce que** le fait de guider une ventilation mécanique selon une pression transpulmonaire surveillée comprend en particulier : le fait d'obtenir une pression transpulmonaire en fin d'expiration selon la pression transpulmonaire surveillée, et de mettre en œuvre une opération de regonflement pulmonaire selon la pression transpulmonaire en fin d'expiration ;
où le fait de mettre en œuvre une opération de regonflement pulmonaire selon la pression transpulmonaire en fin d'expiration comprend en particulier : le fait de guider de sorte à mettre en œuvre l'opération de regonflement pulmonaire ou de directement mettre en œuvre l'opération de regonflement pulmonaire, lorsque la pression transpulmonaire en fin d'expiration est inférieure à un second seuil ; ou durant la mise en œuvre de l'opération de regonflement pulmonaire par un procédé incrémental ou décrémental de pression en fin d'expiration positive, le fait d'arrêter l'opération de regonflement pulmonaire s'il est détecté que la pression transpulmonaire en fin d'expiration est inférieure à un second seuil durant une diminution graduelle de la pression en fin d'expiration positive :
où le procédé comprend en outre le fait de guider un réglage de pression en fin d'expiration positive selon la pression transpulmonaire en fin d'expiration après avoir mis en œuvre l'opération de regonflement pulmonaire.

15. Appareil destiné à mettre en œuvre une surveillance de ventilation de patient sur un dispositif de ventilation anesthésique selon la revendication 12, **caractérisé en ce que** le procédé comprend en outre :
le fait d'acquérir un ou plusieurs éléments parmi une forme d'onde de paramètre, un diagramme de tendance de paramètre ou une valeur de surveillance de paramètre ; et d'envoyer les un ou plusieurs éléments parmi une forme d'onde de paramètre, un diagramme de tendance de paramètre ou une valeur de surveillance de paramètre à une interface d'affichage pour affichage ;
le fait d'acquérir un volume pulmonaire et de générer un graphique cyclique pression-volume qui varie avec un cycle respiratoire; où le graphique cyclique pression-volume comprend un graphique cyclique pression œsophagienne-volume et/ou un graphique cyclique pression transpulmonaire-volume ; ou
le fait de fournir un premier graphique cyclique pression transpulmonaire-volume et un second graphique cyclique pression transpulmonaire-volume à une interface d'affichage d'un affichage pour un affichage superposé après détection qu'une opération de regonflement pulmonaire a commencé ; où le premier graphique cyclique pression transpulmonaire-volume est un graphique cyclique pression transpulmonaire-volume avant l'opération de regonflement pulmonaire, et le second graphique cyclique pression transpulmonaire-volume est un graphique cyclique pression transpulmonaire-volume après l'opération de regonflement pulmonaire.
